# EUROPEAN PATENT SPECIFICATION

(11) **EP 2 214 020 B1**
(45) Date of publication and mention of the grant of the patent: **15.08.2012**
(21) Application number: 09001203.0
(22) Date of filing: 29.01.2009
(51) Int. Cl.: G01N 33/58

(54) **PARP-based cytochemical and histochemical detection methods and kits therefor**
PARP-basierte cytochemische und histochemische Nachweisverfahren und Kits dafür
Procédés de détection histochimique et cytochimique à base de PARP et kits associés

(43) Date of publication of application: 04.08.2010
(73) Proprietor: Bergen Teknologieverforing AS, 5006 Bergen (NO)
(72) Inventor: Ziegler, Mathias, Prof. Dr., 5073 Bergen (NO); Niere, Marc, Dr., 5055 Bergen (NO); Dölle, Christian, Dr., 5063 Bergen (NO)
(74) Representative: Kröncke, Rolf

(56) References cited:
- WO-A-02/44157
- ALVAREZ-GONZALEZ RAFAEL ET AL: "Enzymology of ADP-ribose polymer synthesis" MOLECULAR AND CELLULAR BIOCHEMISTRY, vol. 138, no. 0, 1994, pages 33-37, XP009114379 ISSN: 0300-8177

### SEQUENCE LISTING

<110> Bergen Teknologieverforing AS
<120> PARP-based cytochemical and histocehmical detection methods and kits therefor
<130> 4200-006-EP-1
<160> 2
<170> PatentIn version 3.3
<210> 1
   <211> 4001
   <212> DNA
   <213> human
<220>
   <221> CDS
   <222> (172)..(3213)
<400> 1
   aggcatcagc aatctatcag ggaacggcgg tggccggtgc ggcgtgttcg gtggcggctc     60
   tggccgctca ggcgcctgcg gctgggtgag cgcacgcgag gcggcgaggc ggcagcgtgt    120
   ttctaggtcg tggcgtcggg cttccggagc tttggcggca gctaggggag g atg gcg     177
                                                           Met Ala
                                                           1
   gag tct tcg gat aag ctc tat cga gtc gag tac gcc aag agc ggg cgc      225
   Glu Ser Ser Asp Lys Leu Tyr Arg Val Glu Tyr Ala Lys Ser Gly Arg
           5                   10                  15
   gcc tct tgc aag aaa tgc agc gag agc atc ccc aag gac tcg ctc cgg      273
   Ala Ser Cys Lys Lys Cys Ser Glu Ser Ile Pro Lys Asp Ser Leu Arg
       20                  25                  30
   atg gcc atc atg gtg cag tcg ccc atg ttt gat gga aaa gtc cca cac      321
   Met Ala Ile Met Val Gln Ser Pro Met Phe Asp Gly Lys Val Pro His
   35                  40                  45                  50
   tgg tac cac ttc tcc tgc ttc tgg aag gtg ggc cac tcc atc cgg cac      369
   Trp Tyr His Phe Ser Cys Phe Trp Lys Val Gly His Ser Ile Arg His
                   55                  60                  65
   cct gac gtt gag gtg gat ggg ttc tct gag ctt cgg tgg gat gac cag      417
   Pro Asp Val Glu Val Asp Gly Phe Ser Glu Leu Arg Trp Asp Asp Gln
               70                  75                  80
   cag aaa gtc aag aag aca gcg gaa gct gga gga gtg aca ggc aaa ggc      465
   Gln Lys Val Lys Lys Thr Ala Glu Ala Gly Gly Val Thr Gly Lys Gly
           85                  90                  95
   cag gat gga att ggt agc aag gca gag aag act ctg ggt gac ttt gca      513
   Gln Asp Gly Ile Gly Ser Lys Ala Glu Lys Thr Leu Gly Asp Phe Ala
       100                 105                 110
   gca gag tat gcc aag tcc aac aga agt acg tgc aag ggg tgt atg gag      561
   Ala Glu Tyr Ala Lys Ser Asn Arg Ser Thr Cys Lys Gly Cys Met Glu
   115                 120                 125                 130
   aag ata gaa aag ggc cag gtg cgc ctg tcc aag aag atg gtg gac ccg      609
   Lys Ile Glu Lys Gly Gln Val Arg Leu Ser Lys Lys Met Val Asp Pro
                   135                 140                 145
   gag aag cca cag cta ggc atg att gac cgc tgg tac cat cca ggc tgc      657
   Glu Lys Pro Gln Leu Gly Met Ile Asp Arg Trp Tyr His Pro Gly Cys
               150                 155                 160
   ttt gtc aag aac agg gag gag ctg ggt ttc cgg ccc gag tac agt gcg      705
   Phe Val Lys Asn Arg Glu Glu Leu Gly Phe Arg Pro Glu Tyr Ser Ala
           165                 170                 175
   agt cag ctc aag ggc ttc agc ctc ctt gct aca gag gat aaa gaa gcc      753
   Ser Gln Leu Lys Gly Phe Ser Leu Leu Ala Thr Glu Asp Lys Glu Ala
       180                 185                 190
   ctg aag aag cag ctc cca gga gtc aag agt gaa gga aag aga aaa ggc      801
   Leu Lys Lys Gln Leu Pro Gly Val Lys Ser Glu Gly Lys Arg Lys Gly
   195                 200                 205                 210
   gat gag gtg gat gga gtg gat gaa gtg gcg aag aag aaa tct aaa aaa      849
   Asp Glu Val Asp Gly Val Asp Glu Val Ala Lys Lys Lys Ser Lys Lys
                   215                 220                 225
   gaa aaa gac aag gat agt aag ctt gaa aaa gcc cta aag gct cag aac      897
   Glu Lys Asp Lys Asp Ser Lys Leu Glu Lys Ala Leu Lys Ala Gln Asn
               230                 235                 240
   gac ctg atc tgg aac atc aag gac gag cta aag aaa gtg tgt tca act      945
   Asp Leu Ile Trp Asn Ile Lys Asp Glu Leu Lys Lys Val Cys Ser Thr
           245                 250                 255
   aat gac ctg aag gag cta ctc atc ttc aac aag cag caa gtg cct tct      993
   Asn Asp Leu Lys Glu Leu Leu Ile Phe Asn Lys Gln Gln Val Pro Ser
       260                 265                 270
   ggg gag tcg gcg atc ttg gac cga gta gct gat ggc atg gtg ttc ggt     1041
   Gly Glu Ser Ala Ile Leu Asp Arg Val Ala Asp Gly Met Val Phe Gly
   275                 280                 285                 290
   gcc ctc ctt ccc tgc gag gaa tgc tcg ggt cag ctg gtc ttc aag agc     1089
   Ala Leu Leu Pro Cys Glu Glu Cys Ser Gly Gln Leu Val Phe Lys Ser
                   295                 300                 305
   gat gcc tat tac tgc act ggg gac gtc act gcc tgg acc aag tgt atg     1137
   Asp Ala Tyr Tyr Cys Thr Gly Asp Val Thr Ala Trp Thr Lys Cys Met
               310                 315                 320
   gtc aag aca cag aca ccc aac cgg aag gag tgg gta acc cca aag gaa     1185
   Val Lys Thr Gln Thr Pro Asn Arg Lys Glu Trp Val Thr Pro Lys Glu
           325                 330                 335
   ttc cga gaa atc tct tac ctc aag aaa ttg aag gtt aaa aaa cag gac     1233
   Phe Arg Glu Ile Ser Tyr Leu Lys Lys Leu Lys Val Lys Lys Gln Asp
       340                 345                 350
   cgt ata ttc ccc cca gaa acc agc gcc tcc gtg gcg gcc acg cct ccg     1281
   Arg Ile Phe Pro Pro Glu Thr Ser Ala Ser Val Ala Ala Thr Pro Pro
   355                 360                 365                 370
   ccc tcc aca gcc tcg gct cct gct gct gtg aac tcc tct gct tca gca     1329
   Pro Ser Thr Ala Ser Ala Pro Ala Ala Val Asn Ser Ser Ala Ser Ala
                   375                 380                 385
   gat aag cca tta tcc aac atg aag atc ctg act ctc ggg aag ctg tcc     1377
   Asp Lys Pro Leu Ser Asn Met Lys Ile Leu Thr Leu Gly Lys Leu Ser
               390                 395                 400
   cgg aac aag gat gaa gtg aag gcc atg att gag aaa ctc ggg ggg aag     1425
   Arg Asn Lys Asp Glu Val Lys Ala Met Ile Glu Lys Leu Gly Gly Lys
           405                 410                 415
   ttg acg ggg acg gcc aac aag gct tcc ctg tgc atc agc acc aaa aag      1473
   Leu Thr Gly Thr Ala Asn Lys Ala Ser Leu Cys Ile Ser Thr Lys Lys
       420                 425                 430
   gag gtg gaa aag atg aat aag aag atg gag gaa gta aag gaa gcc aac      1521
   Glu Val Glu Lys Met Asn Lys Lys Met Glu Glu Val Lys Glu Ala Asn
   435                 440                 445                 450
   atc cga gtt gtg tct gag gac ttc ctc cag gac gtc tcc gcc tcc acc      1569
   Ile Arg Val Val Ser Glu Asp Phe Leu Gln Asp Val Ser Ala Ser Thr
                   455                 460                 465
   aag agc ctt cag gag ttg ttc tta gcg cac atc ttg tcc cct tgg ggg      1617
   Lys Ser Leu Gln Glu Leu Phe Leu Ala His Ile Leu Ser Pro Trp Gly
               470                 475                 480
   gca gag gtg aag gca gag cct gtt gaa gtt gtg gcc cca aga ggg aag      1665
   Ala Glu Val Lys Ala Glu Pro Val Glu Val Val Ala Pro Arg Gly Lys
           485                 490                 495
   tca ggg gct gcg ctc tcc aaa aaa agc aag ggc cag gtc aag gag gaa      1713
   Ser Gly Ala Ala Leu Ser Lys Lys Ser Lys Gly Gln Val Lys Glu Glu
       500                 505                 510
   ggt atc aac aaa tct gaa aag aga atg aaa tta act ctt aaa gga gga      1761
   Gly Ile Asn Lys Ser Glu Lys Arg Met Lys Leu Thr Leu Lys Gly Gly
   515                 520                 525                 530
   gca gct gtg gat cct gat tct gga ctg gaa cac tct gcg cat gtc ctg      1809
   Ala Ala Val Asp Pro Asp Ser Gly Leu Glu His Ser Ala His Val Leu
                   535                 540                 545
   gag aaa ggt ggg aag gtc ttc agt gcc acc ctt ggc ctg gtg gac atc      1857
   Glu Lys Gly Gly Lys Val Phe Ser Ala Thr Leu Gly Leu Val Asp Ile
               550                 555                 560
   gtt aaa gga acc aac tcc tac tac aag ctg cag ctt ctg gag gac gac      1905
   Val Lys Gly Thr Asn Ser Tyr Tyr Lys Leu Gln Leu Leu Glu Asp Asp
           565                 570                 575
   aag gaa aac agg tat tgg ata ttc agg tcc tgg ggc cgt gtg ggt acg      1953
   Lys Glu Asn Arg Tyr Trp Ile Phe Arg Ser Trp Gly Arg Val Gly Thr
       580                 585                 590
   gtg atc ggt agc aac aaa ctg gaa cag atg ccg tcc aag gag gat gcc      2001
   Val Ile Gly Ser Asn Lys Leu Glu Gln Met Pro Ser Lys Glu Asp Ala
   595                 600                 605                 610
   att gag cac ttc atg aaa tta tat gaa gaa aaa acc ggg aac gct tgg      2049
   Ile Glu His Phe Met Lys Leu Tyr Glu Glu Lys Thr Gly Asn Ala Trp
                   615                 620                 625
   cac tcc aaa aat ttc acg aag tat ccc aaa aag ttc tac ccc ctg gag      2097
   His Ser Lys Asn Phe Thr Lys Tyr Pro Lys Lys Phe Tyr Pro Leu Glu
               630                 635                 640
   att gac tat ggc cag gat gaa gag gca gtg aag aag ctg aca gta aat      2145
   Ile Asp Tyr Gly Gln Asp Glu Glu Ala Val Lys Lys Leu Thr Val Asn
           645                 650                 655
   cct ggc acc aag tcc aag ctc ccc aag cca gtt cag gac ctc atc aag      2193
   Pro Gly Thr Lys Ser Lys Leu Pro Lys Pro Val Gln Asp Leu Ile Lys
       660                 665                 670
   atg atc ttt gat gtg gaa agt atg aag aaa gcc atg gtg gag tat gag      2241
   Met Ile Phe Asp Val Glu Ser Met Lys Lys Ala Met Val Glu Tyr Glu
   675                 680                 685                 690
   atc gac ctt cag aag atg ccc ttg ggg aag ctg agc aaa agg cag atc     2289
   Ile Asp Leu Gln Lys Met Pro Leu Gly Lys Leu Ser Lys Arg Gln Ile
                   695                 700                 705
   cag gcc gca tac tcc atc ctc agt gag gtc cag cag gcg gtg tct cag     2337
   Gln Ala Ala Tyr Ser Ile Leu Ser Glu Val Gln Gln Ala Val Ser Gln
               710                 715                 720
   ggc agc agc gac tct cag atc ctg gat ctc tca aat cgc ttt tac acc     2385
   Gly Ser Ser Asp Ser Gln Ile Leu Asp Leu Ser Asn Arg Phe Tyr Thr
           725                 730                 735
   ctg atc ccc cac gac ttt ggg atg aag aag cct ccg ctc ctg aac aat     2433
   Leu Ile Pro His Asp Phe Gly Met Lys Lys Pro Pro Leu Leu Asn Asn
       740                 745                 750
   gca gac agt gtg cag gcc aag gtg gaa atg ctt gac aac ctg ctg gac     2481
   Ala Asp Ser Val Gln Ala Lys Val Glu Met Leu Asp Asn Leu Leu Asp
   755                 760                 765                 770
   atc gag gtg gcc tac agt ctg ctc agg gga ggg tct gat gat agc agc     2529
   Ile Glu Val Ala Tyr Ser Leu Leu Arg Gly Gly Ser Asp Asp Ser Ser
                   775                 780                 785
   aag gat ccc atc gat gtc aac tat gag aag ctc aaa act gac att aag     2577
   Lys Asp Pro Ile Asp Val Asn Tyr Glu Lys Leu Lys Thr Asp Ile Lys
               790                 795                 800
   gtg gtt gac aga gat tct gaa gaa gcc gag atc atc agg aag tat gtt     2625
   Val Val Asp Arg Asp Ser Glu Glu Ala Glu Ile Ile Arg Lys Tyr Val
           805                 810                 815
   aag aac act cat gca acc aca cac aat gcg tat gac ttg gaa gtc atc     2673
   Lys Asn Thr His Ala Thr Thr His Asn Ala Tyr Asp Leu Glu Val Ile
       820                 825                 830
   gat atc ttt aag ata gag cgt gaa ggc gaa tgc cag cgt tac aag ccc     2721
   Asp Ile Phe Lys Ile Glu Arg Glu Gly Glu Cys Gln Arg Tyr Lys Pro
   835                 840                 845                 850
   ttt aag cag ctt cat aac cga aga ttg ctg tgg cac ggg tcc agg acc     2769
   Phe Lys Gln Leu His Asn Arg Arg Leu Leu Trp His Gly Ser Arg Thr
                   855                 860                 865
   acc aac ttt gct ggg atc ctg tcc cag ggt ctt cgg ata gcc ccg cct     2817
   Thr Asn Phe Ala Gly Ile Leu Ser Gln Gly Leu Arg Ile Ala Pro Pro
               870                 875                 880
   gaa gcg ccc gtg aca ggc tac atg ttt ggt aaa ggg atc tat ttc gct     2865
   Glu Ala Pro Val Thr Gly Tyr Met Phe Gly Lys Gly Ile Tyr Phe Ala
           885                 890                 895
   gac atg gtc tcc aag agt gcc aac tac tgc cat acg tct cag gga gac     2913
   Asp Met Val Ser Lys Ser Ala Asn Tyr Cys His Thr Ser Gln Gly Asp
       900                 905                 910
   cca ata ggc tta atc ctg ttg gga gaa gtt gcc ctt gga aac atg tat     2961
   Pro Ile Gly Leu Ile Leu Leu Gly Glu Val Ala Leu Gly Asn Met Tyr
   915                 920                 925                 930
   gaa ctg aag cac gct tca cat atc agc aag tta ccc aag ggc aag cac     3009
   Glu Leu Lys His Ala Ser His Ile Ser Lys Leu Pro Lys Gly Lys His
                   935                 940                 945
   agt gtc aaa ggt ttg ggc aaa act acc cct gat cct tca gct aac att     3057
   Ser Val Lys Gly Leu Gly Lys Thr Thr Pro Asp Pro Ser Ala Asn Ile
               950                 955                 960
   agt ctg gat ggt gta gac gtt cct ctt ggg acc ggg att tca tct ggt      3105
   Ser Leu Asp Gly Val Asp Val Pro Leu Gly Thr Gly Ile Ser Ser Gly
           965                 970                 975
   gtg aat gac acc tct cta cta tat aac gag tac att gtc tat gat att      3153
   Val Asn Asp Thr Ser Leu Leu Tyr Asn Glu Tyr Ile Val Tyr Asp Ile
       980                 985                 990
   gct cag gta aat ctg aag  tat ctg ctg aaa ctg  aaa ttc aat ttt        3198
   Ala Gln Val Asn Leu Lys  Tyr Leu Leu Lys Leu  Lys Phe Asn Phe
   995                 1000                 1005
   aag  acc tcc ctg tgg taattgggag aggtagccga gtcacacccg gtggctctgg     3253
   Lys  Thr Ser Leu Trp
   1010
   tatgaattca cccgaagcgc ttctgcacca actcacctgg ccgctaagtt gctgatgggt    3313
   agtacctgta ctaaaccacc tcagaaagga ttttacagaa acgtgttaaa ggttttctct    3373
   aacttctcaa gtcccttgtt ttgtgttgtg tctgtgggga ggggttgttt tggggttgtt    3433
   tttgtttttt cttgccaggt agataaaact gacatagaga aaaggctgga gagagattct    3493
   gttgcataga ctagtcctat ggaaaaaacc aagcttcgtt agaatgtctg ccttactggt    3553
   ttccccaggg aaggaaaaat acacttccac ccttttttct aagtgttcgt ctttagtttt   3613
   gattttggaa agatgttaag catttatttt tagttaaaaa taaaaactaa tttcatacta    3673
   tttagatttt cttttttatc ttgcacttat tgtccccttt ttagtttttt ttgtttgcct   3733
   cttgtggtga ggggtgtggg aagaccaaag gaaggaacgc taacaatttc tcatacttag    3793
   aaacaaaaag agctttcctt ctccaggaat actgaacatg ggagctcttg aaatatgtag    3853
   tattaaaagt tgcatttgaa attcttgact ttcttatggg cacttttgtc ttccaaatta    3913
   aaactctacc acaaatatac ttacccaagg gctaatagta atactcgatt aaaaatgcag    3973
   atgccttctc taaaaaaaaa aaaaaaaa                                       4001
<210> 2
   <211> 1014
   <212> PRT
   <213> human
<400> 2
   Met Ala Glu Ser Ser Asp Lys Leu Tyr Arg Val Glu Tyr Ala Lys Ser
   1               5                   10                  15
   Gly Arg Ala Ser Cys Lys Lys Cys Ser Glu Ser Ile Pro Lys Asp Ser
               20                  25                  30
   Leu Arg Met Ala Ile Met Val Gln Ser Pro Met Phe Asp Gly Lys Val
           35                  40                  45
   Pro His Trp Tyr His Phe Ser Cys Phe Trp Lys Val Gly His Ser Ile
       50                  55                  60
   Arg His Pro Asp Val Glu Val Asp Gly Phe Ser Glu Leu Arg Trp Asp
   65                  70                  75                  80
   Asp Gln Gln Lys Val Lys Lys Thr Ala Glu Ala Gly Gly Val Thr Gly
   85 90 95
   Lys Gly Gln Asp Gly Ile Gly Ser Lys Ala Glu Lys Thr Leu Gly Asp
   100 105 110
   Phe Ala Ala Glu Tyr Ala Lys Ser Asn Arg Ser Thr Cys Lys Gly Cys
   115 120 125
   Met Glu Lys Ile Glu Lys Gly Gln Val Arg Leu Ser Lys Lys Met Val
   130 135 140
   Asp Pro Glu Lys Pro Gln Leu Gly Met Ile Asp Arg Trp Tyr His Pro
   145 150 155 160
   Gly Cys Phe Val Lys Asn Arg Glu Glu Leu Gly Phe Arg Pro Glu Tyr
   165 170 175
   Ser Ala Ser Gln Leu Lys Gly Phe Ser Leu Leu Ala Thr Glu Asp Lys
   180 185 190
   Glu Ala Leu Lys Lys Gln Leu Pro Gly Val Lys Ser Glu Gly Lys Arg
   195 200 205
   Lys Gly Asp Glu Val Asp Gly Val Asp Glu Val Ala Lys Lys Lys Ser
   210 215 220
   Lys Lys Glu Lys Asp Lys Asp Ser Lys Leu Glu Lys Ala Leu Lys Ala
   225 230 235 240
   Gln Asn Asp Leu Ile Trp Asn Ile Lys Asp Glu Leu Lys Lys Val Cys
   245 250 255
   Ser Thr Asn Asp Leu Lys Glu Leu Leu Ile Phe Asn Lys Gln Gln Val
   260 265 270
   Pro Ser Gly Glu Ser Ala Ile Leu Asp Arg Val Ala Asp Gly Met Val
   275 280 285
   Phe Gly Ala Leu Leu Pro Cys Glu Glu Cys Ser Gly Gln Leu Val Phe
   290 295 300
   Lys Ser Asp Ala Tyr Tyr Cys Thr Gly Asp Val Thr Ala Trp Thr Lys
   305 310 315 320
   Cys Met Val Lys Thr Gln Thr Pro Asn Arg Lys Glu Trp Val Thr Pro
   325 330 335
   Lys Glu Phe Arg Glu Ile Ser Tyr Leu Lys Lys Leu Lys Val Lys Lys
   340 345 350
   Gln Asp Arg Ile Phe Pro Pro Glu Thr Ser Ala Ser Val Ala Ala Thr
   355 360 365
   Pro Pro Pro Ser Thr Ala Ser Ala Pro Ala Ala Val Asn Ser Ser Ala
   370 375 380
   Ser Ala Asp Lys Pro Leu Ser Asn Met Lys Ile Leu Thr Leu Gly Lys
   385 390 395 400
   Leu Ser Arg Asn Lys Asp Glu Val Lys Ala Met Ile Glu Lys Leu Gly
   405 410 415
   Gly Lys Leu Thr Gly Thr Ala Asn Lys Ala Ser Leu Cys Ile Ser Thr
   420 425 430
   Lys Lys Glu Val Glu Lys Met Asn Lys Lys Met Glu Glu Val Lys Glu
   435 440 445
   Ala Asn Ile Arg Val Val Ser Glu Asp Phe Leu Gln Asp Val Ser Ala
   450 455 460
   Ser Thr Lys Ser Leu Gln Glu Leu Phe Leu Ala His Ile Leu Ser Pro
   465 470 475 480
   Trp Gly Ala Glu Val Lys Ala Glu Pro Val Glu Val Val Ala Pro Arg
   485 490 495
   Gly Lys Ser Gly Ala Ala Leu Ser Lys Lys Ser Lys Gly Gln Val Lys
   500 505 510
   Glu Glu Gly Ile Asn Lys Ser Glu Lys Arg Met Lys Leu Thr Leu Lys
   515 520 525
   Gly Gly Ala Ala Val Asp Pro Asp Ser Gly Leu Glu His Ser Ala His
   530 535 540
   Val Leu Glu Lys Gly Gly Lys Val Phe Ser Ala Thr Leu Gly Leu Val
   545 550 555 560
   Asp Ile Val Lys Gly Thr Asn Ser Tyr Tyr Lys Leu Gln Leu Leu Glu
   565 570 575
   Asp Asp Lys Glu Asn Arg Tyr Trp Ile Phe Arg Ser Trp Gly Arg Val
   580 585 590
   Gly Thr Val Ile Gly Ser Asn Lys Leu Glu Gln Met Pro Ser Lys Glu
   595 600 605
   Asp Ala Ile Glu His Phe Met Lys Leu Tyr Glu Glu Lys Thr Gly Asn
   610 615 620
   Ala Trp His Ser Lys Asn Phe Thr Lys Tyr Pro Lys Lys Phe Tyr Pro
   625 630 635 640
   Leu Glu Ile Asp Tyr Gly Gln Asp Glu Glu Ala Val Lys Lys Leu Thr
   645 650 655
   Val Asn Pro Gly Thr Lys Ser Lys Leu Pro Lys Pro Val Gln Asp Leu
   660 665 670
   Ile Lys Met Ile Phe Asp Val Glu Ser Met Lys Lys Ala Met Val Glu
   675 680 685
   Tyr Glu Ile Asp Leu Gln Lys Met Pro Leu Gly Lys Leu Ser Lys Arg
   690 695 700
   Gln Ile Gln Ala Ala Tyr Ser Ile Leu Ser Glu Val Gln Gln Ala Val
   705 710 715 720
   Ser Gln Gly Ser Ser Asp Ser Gln Ile Leu Asp Leu Ser Asn Arg Phe
   725 730 735
   Tyr Thr Leu Ile Pro His Asp Phe Gly Met Lys Lys Pro Pro Leu Leu
   740 745 750
   Asn Asn Ala Asp Ser Val Gln Ala Lys Val Glu Met Leu Asp Asn Leu
   755 760 765
   Leu Asp Ile Glu Val Ala Tyr Ser Leu Leu Arg Gly Gly Ser Asp Asp
   770 775 780
   Ser Ser Lys Asp Pro Ile Asp Val Asn Tyr Glu Lys Leu Lys Thr Asp
   785 790 795 800
   Ile Lys Val Val Asp Arg Asp Ser Glu Glu Ala Glu Ile Ile Arg Lys
   805 810 815
   Tyr Val Lys Asn Thr His Ala Thr Thr His Asn Ala Tyr Asp Leu Glu
   820 825 830
   Val Ile Asp Ile Phe Lys Ile Glu Arg Glu Gly Glu Cys Gln Arg Tyr
   835 840 845
   Lys Pro Phe Lys Gln Leu His Asn Arg Arg Leu Leu Trp His Gly Ser
   850 855 860
   Arg Thr Thr Asn Phe Ala Gly Ile Leu Ser Gln Gly Leu Arg Ile Ala
   865 870 875 880
   Pro Pro Glu Ala Pro Val Thr Gly Tyr Met Phe Gly Lys Gly Ile Tyr
   885 890 895
   Phe Ala Asp Met Val Ser Lys Ser Ala Asn Tyr Cys His Thr Ser Gln
   900 905 910
   Gly Asp Pro Ile Gly Leu Ile Leu Leu Gly Glu Val Ala Leu Gly Asn
   915 920 925
   Met Tyr Glu Leu Lys His Ala Ser His Ile Ser Lys Leu Pro Lys Gly
   930 935 940
   Lys His Ser Val Lys Gly Leu Gly Lys Thr Thr Pro Asp Pro Ser Ala
   945 950 955 960
   Asn Ile Ser Leu Asp Gly Val Asp Val Pro Leu Gly Thr Gly Ile Ser
   965 970 975
   Ser Gly Val Asn Asp Thr Ser Leu Leu Tyr Asn Glu Tyr Ile Val Tyr
   980 985 990
   Asp Ile Ala Gln Val Asn Leu Lys Tyr Leu Leu Lys Leu Lys Phe Asn
   995 1000 1005
   Phe Lys Thr Ser Leu Trp
   1010

## Description

The present invention relates to new cytochemical and histochemical detection methods based on the enzymatic activity of poly-ADP-ribose-polymerasse (PARP). In particular, the present invention provides methods and systems for cytochemical or histochemical detection of a molecule of interest based on detecting poly-ADP-ribose (PAR). Further, the present invention provides methods for screening for compounds altering the amount of NAD or analogs thereof in subcellular compartments based on detecting the amount of PAR formed in said subcellular compartment as well as methods for determining the spatial distribution of a molecule of interest. In a further aspect, the present invention provides systems and kits for conducting said methods as well as a new expression vectors suitable for said systems.

### Background of the Invention

Biological polymers (biopolymers) are made of repetitive units, namely monomers. They are generated from relatively small molecules by enzymes such as polymerases or synthases. Polymers are composed of either different monomer entities (heterogeneous polymers) or a single type of monomeric units (homogenous polymers). Polymer generation is a common process in organisms of all natural kingdoms (bacteria, archaea, fungi, plants and animals). Biological polymers can be synthesized from different substance classes.

Polymer formation is known for various small molecules, like sugar molecules. For example, nicotinamide adenine dinucleotide (NAD) serves as substrate for poly-ADP-ribose-polymerases (PARPs), an enzyme class which cleaves the nicotinamide ring from the NAD molecules and generates polymers of the remaining ADP ribose units. For instance, the mammalian PARP enzymes are capable of generating PAR consisting of up to 200 ADP-ribose units and to attach them to specific acceptor proteins including themselves (automodification).

In general, biopolymers can consist of several hundreds to thousand individual subunits and can be branched providing the polymer with a characteristic three-dimensional structure.

Typically, the catalyzing enzymes differ in their subcellular localization. Eukaryotic organelles contain partial or complete metabolic pathways. While glycogen synthesis takes place in the cytosol and the products are stored in granular structures, starch synthesis in plants takes place within specific membrane coated organelles (plastids). Other polymerases, like DNA and RNA polymerases, exhibit their catalytic activity in specific compartments, e.g. in the nucleus, or in the mitochondria and, in plants, in the chloroplasts. The above mentioned PARPs exist in several isoforms located in different cellular compartments. In humans, the most abundant and active form, PARP1, is located to the nucleus. The distinct locations of polymerases and synthases also indicate a compartmented availability of the substrates needed for the reaction.

Antibody-based detection systems are important and prevalent methods for the specific detection of macromolecules such as proteins or biopolymers. For instance, immunoblot analyses or enzyme-linked immunosorbent assays (ELISA) of samples deriving from tissues, cells, body fluids are currently used both in molecular biology and biochemistry as well as in diagnostics to e.g. determine the abundancy of proteins, their modification state and changes in gene expression levels.

Detection is usually performed by binding of a secondary antibody that specifically detects the primary antibody bound to the antigen of interest (AOI). Most often, the secondary antibody is conjugated with an enzyme activity (e.g., alkaline phosphatase or horse radish peroxidase). This enzyme activity can be used to convert a soluble dye into an insoluble form of e.g. a different color (colorimetric detection). The more popular detection method using enzyme-conjugated secondary antibodies is the incubation with a substrate that subsequently emits light (chemiluminescent detection), which is detected either by a CCD camera that displays an image in dependence of the amount of light generated or by placing a membrane onto a film. Besides colorimetric or chemiluminescent techniques, antibodies can be conjugated with fluorescent dyes for antigen detection. Here, particularly fluorophores emitting near-infrared light have been shown to be most suitable, since membrane surfaces and biomolecules exhibit greatly reduced autofluorescence in this wavelength range.

The applicability of immunoblot analyses and ELISA is limited by the level of detection, e.g. for the identification of low abundant antigens (such as hormones) in body fluids (e.g., blood). The detection limit of proteins using fluorescent and chemiluminescent techniques can be up to the lower picogram range.

Other immunochemical techniques such as immunocytochemistry and immunohistochemistry are commonly applied to narrow down the location of the AOI in tissues or cells. Immunodetection, which is predominantly done by using enzyme-conjugated or fluorescence-labelled antibodies, allows for analyzing subcellular localization of the AOI (e.g., its association with a subcellular compartment). However, it does not enable the determination of its suborganellar distribution. The identification of silver enhanced immunogold particles by electron microscopy is a reliable antibody-based detection method that overcomes this difficulty.

The generation of antibodies using recombinant DNA technologies is impeded because of their complex molecular architecture: Antigen binding by a classical vertebrate antibody requires the variable domains of both a heavy chain and a light chain. As an important exception, antibodies from camelid and shark species were shown to lack the light chain, thus, forming single chain antibodies. The heavy chain is made up of two constant domains (CH2 and CH3) and a single variable domain (VHH), which is important for antigen binding. Due to the requirements of only one polypeptide chain to form a functional immunoglobin, these antibodies are considered a powerful tool in both therapeutic concepts and applied research, since they are suitable for a manipulation using recombinant DNA technologies and can therefore be expressed as multispecific and multifunctional fusion proteins in heterologous systems.

Knowing the precise subcellular localization of a protein is essential for the understanding of its biological function. In this regard, it is of importance to establish whether a protein is present within a specific, membrane-enclosed cell organelle. A particular challenge represent organelles with more than one surrounding membrane, such as mitochondria. Here, soluble or peripheral membrane proteins could reside in the matrix, the intermembrane space or at the outer surface (of the outer membrane). There are various methods to visualize the selective association of a molecule of interest (MOI), such as a protein of interest (POI) with subcellular compartments. These are most commonly based on overexpressed fusion proteins (part of which is the POI). One of the most widely used applications is the fusion of the POI to a fluorescent protein, such as the green fluorescent protein (GFP), which is then detected by its intrinsic fluorescence. Alternative detection methods use immunochemical techniques or specific properties of the protein or peptide fused to the POI (such as a catalytic activity). A general disadvantage of these methods consists in the lack of resolution of the suborganellar localization.

That is, these methods mostly rely on (fluorescence) microscopic analyses which do not allow establishing whether the POI is located within the organelle or, for example, associated at the outer surface. At present, immunogold labelling in conjunction with electron microscopy is used to resolve this problem. Alternatively, cell or tissue disintegration and subfractionation of organelles are used to address this problem. However, these methods are labour-intensive and often inconclusive. Moreover, they are not applicable to large scale analyses.

Analyses of metabolites represent an important part of diagnostic procedures, but are also a vital instrument to understand the basic functions and advanced signalling pathways in cells. At present, comprehensive determinations of cellular, tissue or organismal metabolites are conducted to evaluate metabolite changes (metobolomics). These investigations use different methods (mass spectrometry coupled to chromatographic procedures, NMR and others), but do largely not consider subcellular metabolite pools. This limitation is, in part, caused by the necessity to obtain pure subcellular fractions which is time-consuming and error-prone.

Still, the vast majority of current knowledge regarding the subcellular distribution of small molecules (metabolites) has been established by cell fractionation and subsequent analysis of metabolites in the enriched organelle fractions. Owing to the more advanced methods of subcellular protein localization, the presence of metabolites has also been inferred from the localization of enzymes specifically converting them. Only in rare instances can metabolites be directly detected in their physiological compartments. For example, reduced pyridine nucleotides are fluorescent and they can be visualized in live cells by advanced microscopic techniques. While advanced physical methods (e.g., NMR) allow to specifically detect metabolites and their relative changes in cells or tissues, their resolution is still insufficient to resolve metabolite concentrations in subcellular structures.

Enzymes have been used as biosensors to detect metabolites, primarily in fluids, by so called enzyme based self-referencing biosensors. Despite the ongoing minimization of such devices, they are not applicable to the study of subcellular metabolite pools. In particular, glucose detection devices are of interest to monitor blood levels in diabetes patients. Based on chemical conversions, optical methods have been proposed which result in the formation of a fluorescent glucose adduct that can be detected.

In order to detect biopolymers (e.g., in biological samples), different methods can be applied. Immunochemical detection allows for specific and sensitive identification of a given macromolecule using specifically raised antibodies. The antibodies do not detect the polymer components in their monomeric state. Alternatively, the polymers can be detected by chemical modification of reactive groups of the monomeric subunits. While the use of polymerases has several technical applications, their utility as molecular detectors, for example, in cell based systems, has not been exploited. Being substrate for pomymerase, in principle, the presence of the monomers within a system can be deduced from their occurrence in polymers.

Hence, there is ongoing need for new methods and tools allowing detection of a molecule of interest (MOI), like an AOI or POI, in a probe based on cytochemical or histochemical detection systems. In particular, there is ongoing need for histochemical and cytochemical analysis, in particular of immunocytochemical and immunohistochemical analysis of samples, like biological samples, allowing qualitive as well as quantative determination of the molecule of interest with increased sensitivity of the detection method.

### Brief description of the present invention

In a first aspect, the present invention provides a method for cytochemical or histochemical detection of a molecule of interest, comprising the steps of
a. providing a molecule having a poly-ADP-ribose-polymerase (PARP) activity linked with a molecule able to bind directly or indirectly to the molecule of interest, or nucleid acids encoding the same;
b. allowing binding of the molecule of a.) to the molecule of interest;
c. providing a substrate of the PARP enzyme and generation of poly-ADP-ribose (PAR) by enzymatic reaction of the PARP enzyme;
d. detection of PAR.

In a further aspect, the present invention provides methods for screening for compounds altering the amounts of substrates of PARP, like NAD or analogs thereof, In subcellular compartments comprising the use of a fusion construct of a catalytic moiety of PARP and a moiety directing the catalytic moiety of PARP to a predetermined subcellular compartment and determining the amount of PAR formed in a subcellular compartment of cells transfected with the fusion construct.

In addition, the present invention relates to a method for determining the spatial distribution of a molecule of interest in a cell based on the synthesis of PAR. Moreover, the present invention provides expression vectors comprising the catalytic domain of PARP downstream of a multiple cloning site allowing introduction of the molecule of interest into the multiple cloning site. Finally, the present invention relates to systems or kits for analysing the subcellular localization of metabolites as well as for cytochemical or histochemical detection of a molecule of interest comprising the use of a vector encoding the catalytic domain of PARP downstream of a molecule for targeting said catalytic domain of PARP to the predetermined subcellular compartment and/or a molecule allowing to bind to the molecule of interest.

Finally, the use of the PARP enzyme in cytochemical or histochemical detection of a molecule of interest is disclosed.

### Brief description of the drawings

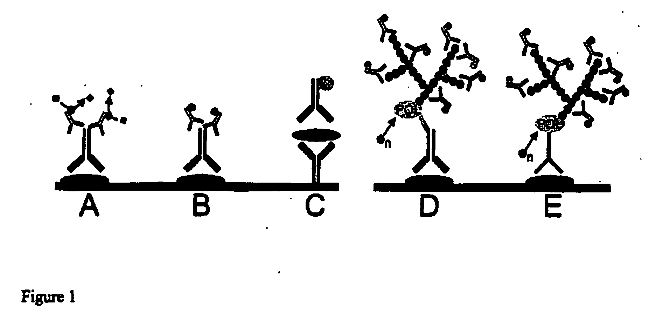

Figure 1 is a scheme showing the immunodetection principle and the improved sensitivity by enzymatically generated polymers according to the present invention. A: Immunodetection by an enzyme coupled to the secondary antibody, generating a colorful product or light, chemiluminescence. B: Immunodetection by a fluorophor coupled to the secondary antibody. C: Immunodetection of a target molecule by an immobilized antibody, followed by the binding of a fluorophor conjugated secondary antibody. D: Immunodetection of a biopolymer generated by a polymerase bound to the primary antibody, for example, via a biotin/streptavidin interaction. E: Immunodetectin of biopolymer generated by a polymerase as a part of a chimeric fusion protein, consisting of the polymerase activity and a single polypeptide chain antibody.

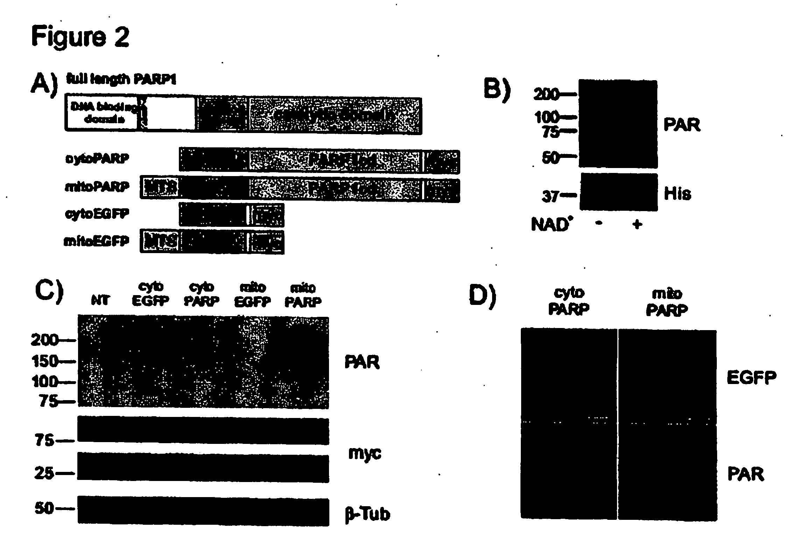

Figure 2 demonstrates that the catalytic domain of PARP 1 is active in vitro and in cells:
   Figure 2A: Molecular architecture of poly-ADP-ribose-polymerase 1 and EGFP fusion constructs. MTS, mitochondrial targeting sequence. Figure 2B: Purified PARP 1 catalytical domain (amino acids 652 to 1014) expressed in E. coli was incubated with 1 mM NAD+. Automodification of PARP1cd was detected by Western blot analysis using an antibody specific for PAR. Figure 2C: Western blot analysis using an antibody specific for PAR after transient expression of cytosolic and mitochondrial EGFP (cytoEGFP and mitoEGFP, respectively) and EGFP-PARP1cd (cytoPARP1cd and mitoPARP1cd, respectively) fusion constructs in HeLaS3 cells reveals accumulation of PAR in cells expressing mitochondrial PARP1cd fusion proteins. Figure 2D: Images showing HeLaS3 cells subjected to PAR immunocytochemistry 24 hours after transient transfection with mitoPARP- or cytoPARP-encoding vectors.

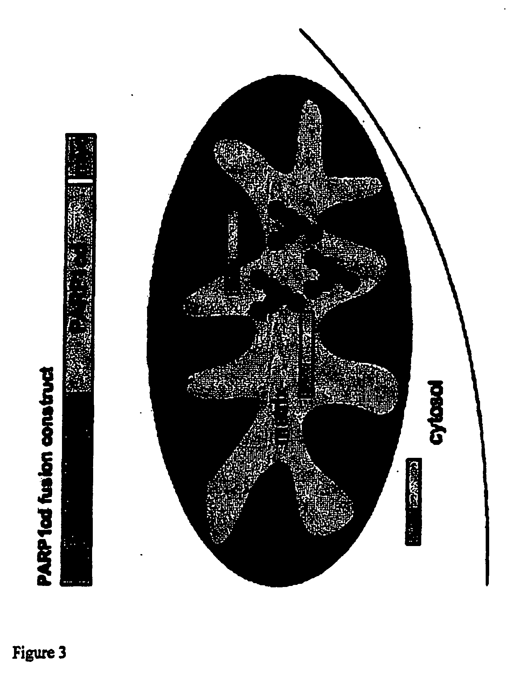

Figure 3; Schematic overview of the biochemical localization approach. The PARP1cd fusion construct consists of the C-terminal 443 amino acid residues of full-length PARP 1 harbouring the catalytic domain C-terminally fused to a protein of interest (upper panel). Differently targeted PARP1cd fusion constructs give rise to distinguishable levels of PAR accumulation (lower panel). While no PAR accumulation is detectable for cytosolic fusion proteins, mitochondrial matrix localized proteins generate PAR. Levels of PAR at the lower detection limit (dotted lines) may be obtained from proteins situated in the intermembrane space of mitochondria.

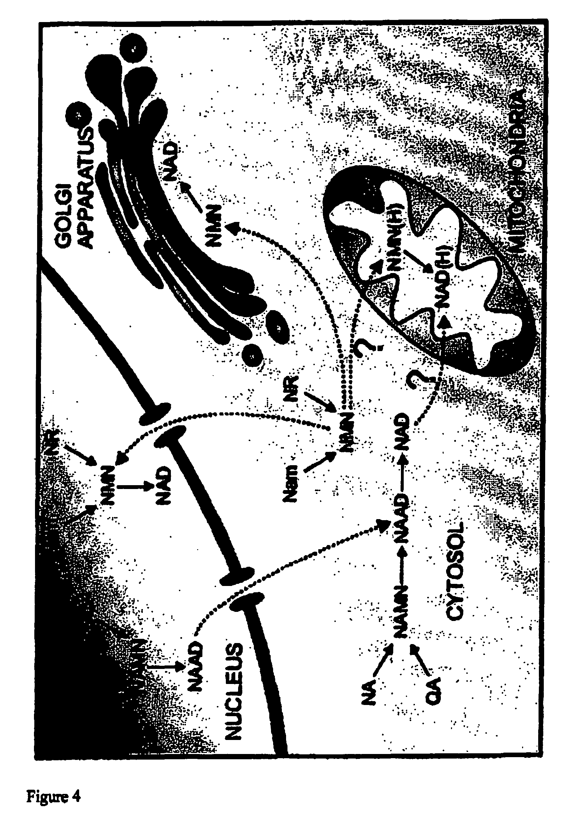

Figure 4: Subcellular compartimentalization of NAD biosynthesis The subcellular distribution of all known steps of human NAD biosynthesis as revealed herein is summarized. The majority of NAD biosynthetic enzymes localize distributed throughout the nucleus and cytosol, allowing complete pathways to occur, NAD synthetase appears to be exclusively expressed in the cytosol. Thus, amidation of NAAD to NAD is only possible in this compartment. The human NMNAT isoforms show the most variable distribution pattern, including nucleus (NMNAT1) Golgi apparatus association (NMNAT2) and mitochondrial matrix (NMNAT3). All reactions (arrows) and intermediates that may occur in the depicted organelles are indicated. Possible import of substrates for mitochondrial NAD synthesis is indicated by dashed lines.

### Detailed description of the present invention

In a first aspect, the invention is directed to a method for cytochemical or histochemical detection of a molecule of interest comprising the steps of
a. providing a molecule having a poly-ADP-ribose-polymerase (PARP) activity linked with a molecule able to bind directly or indirectly to the molecule of interest, or nucleic acids encoding the same;
b. allowing binding of the molecule of a.) to the molecule of interest;
c. providing a substrate of the PARP enzyme and generation of poly-ADP-ribose (PAR) by enzymatic reaction of the PARP enzyme,
d. detection of PAR.

With the term "able to bind directly or indirectly to the molecule of interest" is meant herein that the PARP activity may be bound directly to the binding partner of the MOI, e.g. an antibody directed against the MOI, whereby the PARP activity is covalently bound to the antibody. Alternatively, indirect binding may be given in case where a primary antibody directed aginst the MOI is used and said primary antibody is detected with a secondary antibody having a PARP activity linked thereto.

Of course, steps b. and c. above may be conducted vice versa, namely, the synthesis of the polymer PAR takes places first and, thereafter, the binding of the molecule of step a. to the MOI is enabled.

In a further aspect, the present invention is related to a method for screening for compounds altering the amounts of NAD or analogs thereof in subcellular compartments comprising the steps of:
a. providing a fusion construct of a catalytic moiety of PARP and a moiety directing the catalytic moiety of PARP to a predetermined subcellular compartment;
b. providing a cell system containing the compounds of interest;
c. introducing the fusion construct into the cell system
d. determining the amount of PAR formed in the subcellular compartment in cells of the cell systems.

Moreover, in another aspect, the present invention is directed to a method for determining the spatial distribution of a molecule of interest, comprising the steps of:
a. providing a fusion construct comprising or encoding the catalytic domain of PARP and the molecule of interest;
b. introducing the fusion construct into a cell;
c. determining the localization of the fusion construct based on the detection of generated PAR.

In addition, the present invention is directed to a method for cytochemical or histochemical detection of a molecule of interest comprising the steps of:
a. providing a molecule having a PARP activity linked with a molecule able to bind directly or indirectly to the molecule of interest;
b. allowing binding of the molecule of a.) with the molecule of interest;
c. generation and detection of PAR.

As used herein, the term "NAD or analogs thereof" refer to substrates of the poly-ADP-ribose-polymerase resulting in the generation of oligomers or polymers of the substrate analogously to the generaton of poly-ADP-ribose when using NAD as a substrate.

Further, the term PAR includes PAR analogs obtainable when using NAD analogs as substrate for PARP.

That is, the present inventors recognized that the enzyme poly-ADP-ribose-polmyerase (PARP), like the human PARP, e.g. the human PARP 1, represents a powerful molecular detection tool in generating polymers of poly-ADP-ribose (PAR) which eventually are detected by known techniques, like immunochemical, chemical or physical detection methods. The synthesis of the PAR based on the PARP enzymatic activity allows to amplify a signal of the PARP molecule or any moiety linked with the PARP molecule since the polymer provides multiple sites for the binding of detector molecules like polymer recognizing antibodies or multiples sites having chemically attached fluorophores or other marker moieties.

Thus, the underlying principle allows to amplify signals and, hence, to strongly exceed the sensitivity of detection methods, in particular, histochemical and cytochemical detection methods. Thus, the present system and methods based on PARP enzymatic activity and PAR detection represents a powerful alternative for known enzyme based detection methods, like systems based on alkaline phosphotase or peroxidase.

As used herein, the term "having a poly-ADP-ribose-polymerase (PARP) activity" refers to a molecule, typically a polypeptide having at least the catalytic moiety of PARP capable of generating PAR, for example, the human PARP1 catalytic domain, aa 652 to aa 1014 of Seq. ID No. 1 or Seq. ID No. 2 derived from GeneBank Acc. No. NP 001618. The catalytic moiety of PARP allows to synthesize poly-ADP-ribose from the substrate NAD or analogs thereof, e.g. 1, N⁸-etheno NAD, 3-acetylpyridine adenine dinucleotide (Oei et al, FEBS Lett. 397, pp 17-21, 1996) or biotinylated NAD. The PARP molecule may be provided in form of a polypeptide or in form of a nucleic acid sequence encoding the polypeptide.

The term "molecule able to bind to the molecule of interest" refers to a binding agent allowing to bind to the molecule of interest, typically, via non-covalent forces. Typical examples of said molecules comprise antibodies, like polyclonal, monoclonal antibodies or single chain antibodies, or polypeptides derived from antibodies. Typically, said binding agent interacts specifically with the molecule of interest. Alternatively, the molecule able to bind to the molecule of interest may be ligand or binding partner of the moiety of interest. For example, in case that receptor molecules represent the moiety of interest, the corresponding binding partner is a ligand of said receptor.

Further, the term "moiety directing the catalytic moiety of PARP to a predetermined subcellular compartment" refers to signal sequences known in the art for targeting molecules, typically polypeptides to specific subcellular compartments. For example, said moiety is the mitochondrial targeting sequence derived from the human cytochrome C oxidase subunit 8A, GeneBank Acc. No. NP004065.

In a preferred embodiment, the PARP enzymatic activity is linked with a binding partner, like a monoclonal antibody or a single chain antibody. Preferred, the PARP is attached covalently to the binding partner. In another preferred embodiment, the binding agent of the molecule of interest is a natural ligand of the moiety of interest. In another preferred embodiment, the PARP moiety is linked with avidin or streptavidin and, in addition, the antibody, like a monoclonal antibody or a single chain antibody, is labelled with biotin (or vice versa), thus, interaction between the biotin streptavidin (avidin) molecules occurs connecting the PARP activity with the antibody. Alternatively, the fusion construct comprises a sequence encoding streptavidin or avidin.

The PARP allows the generation of the poly-ADP-ribose (PAR) polymer. The PAR polymer is typically covalently attached to the enzyme and, consequently, allows a detailed and very specific detection of the spatial distribution of the enzyme and, thus, of the molecule of interest.

As used herein, the molecule of interest includes in particular polypeptides and nucleic acid molecules. Preferably, the molecule of interest (MOI) is a polypeptide or peptide of interest (POI), in particular, an antigen of interest (AOI).

Combining the molecule of interest or, in particular, the peptide of interest, with the catalytic activity of PARP allows to determine the spatial distribution of said molecule of interest due to detection of the presence of PAR formed due to the enzymatic activity of PARP.

Additionally, for determining the spatial distribution of the molecule of interest, a fusion construct comprising the catalytic domain of PARP and the molecule of interest in form of a vector is introduced into cells. Of course, the fusion construct may already be in form of a polypeptide which may be introduced into the cells by known techniques.

After translation of the nucleic acid sequence introduced with an expression vector containing the fusion construct in case of nucleic acids, or of the fused product of the molecule of interest in the catalytic domain of PARP in form of a polypeptide, the localisation of said fused product may be determined based on PAR generation.

The fusion construct may further contain a signalling or targeting sequence allowing site specific targeting of the construct, e.g. to an organelle of the cell. These vectors may allow to manipulate NAD levels on the subcellular level. For example, a targeted expression of PARP to peroxisomes or to the ER leads to compartment specific PAR formation as well.

In contrast to current methods which determine the subcellular localisation of a POI based on the utilisation of fluorescent proteins fused to the POI and applying immunocytochemistry or subcellular fractionation, the present invention allows to determine the subcellular/suborganellar protein localisation in more detail and with less amounts of fusion product. In contrast to methods known in the art, the present method allows to determine the suborganellar localization and would not require the utilisation of cost intensive instrumentation. Based on the overexpression of a fusion protein consisting of the POI and a known polymerase protein, it is possible to amplify the signal in the presence of the substrate of the polymerase whereby the substrate may be derived from endogenous or exogenous sources, thus, allowing the detection with higher sensitivity. The polymerase activity is directed to the natural subcellular compartment of the POI. The differential presence of metabolites serving as the substrate for enzymatic generation of polymers in subcellular structures or the differential presence of enzyme activities that degrade the polymers determine whether the overexpression of the fusion construct will lead to the formation of detectable polymers. The specificity may depend on the endogenous conditions of the model system and can be modulated for example by targeted overexpression or down regulation of degrading enzymes. For example, in case of the poly-ADP-ribose glycohydrolase (PARG), which represents the enzyme responsible for degradation of PAR, an inhibitor of this enzyme may be used to inhibit any degradation of PAR.

The determination of the localisation fusion construct is achieved by the detection of the polymerase product, like PAR, generated in the living system by the polymerase reaction. Detection is achieved by known techniques, for example, by polymer specific antibodies, Whether or not detectable polymers can be formed in a specific sub-compartment is tested by overexpression of the polymerase activity endowed with a known targeting sequence directing the protein to the subcellular structure of interest.

The method and system for conducting said method is particularly applicable for identifying protein localization within membrane coated subcellular structures. For large scale approaches, a library of POI-PARP fusion constructs can be constructed and used to screen for subcellular localization of a large set of proteins.

In addition, overexpression of a PARG activity will reduce the levell of PAR if the PARG construct is directed to the same subcellular structure as the POI-PARP fusion construct.

In another embodiment, the present invention relates to a method for screening for compounds altering the amounts of NAD in subcellular compartments. Said method comprises the steps of providing a fusion construct of the catalytic moiety of PARP and a second moiety linked therewith, whereby said second moiety allows directing the catalytic moiety of PARP to a predetermined subcellular compartment. When transfecting cells with said fusion construct, for example a vector as defined herein, cells are transfected and the fusion construct is expressed as a fused protein. The PARP moiety will start to polymerize ADP-ribose from its substrate NAD when sufficient NAD is present in the subcellular or suborganellar compartment, thus, producing detectable PAR molecules. In case a compound modulating NAD metabolism is introduced into said system, alteration of the amounts of NAD, and consequently of formed PAR will occur, thus, allowing to identify said compounds.

Since the presence of PAR also depends on the activity of PARG, the method is equally sensitive to detect compounds modulating PARG activity in a compartment-specific manner.

Thus, an assay based on the above method is provided allowing screening of new molecules which may be useful for treating or preventing diseases wherein NAD, PARG or PAR and the PARP enzyme are involved.

Since the polymer detection is done typically by using polymer-specific antibodies or other agents which specifically interact with the polymers, signal intensities correlate with the concentrations of the metabolite of interest, e.g. NAD. Accordingly, the method and the assay permit to reveal changes of subcellular metabolite concentrations, e.g. NAD concentrations, brought about by changes of the physiological state of the model system. Thus, it is possible to use the assay representing a simple and fast but also reliable method for identifying compounds altering the NAD pool or the activity of PARP enzyme. PARP is known to be involved in apoptosis and in DNA repair, hence, inhibitors or compounds altering the activity of PARP may be useful for inducing apoptosis in cells, in particular, cancerous cells. Hence, said inhibitors may be useful for the treatment of cancer or other diseases involving DNA damage or abnormal proliferation of cells.

In a further aspect, the present invention relates to a system, kit or assay for analysing subcellular localization of metabolites comprising a vector comprising a nucleic acid sequence encoding the catalytic domain of PARP operably linked with a molecule allowing targeting of the catalytic domain of PARP to the subcellular compartment and means for the detection of PAR formed due to PARP activity. Optionally, the kit further comprises a vector containing a nucleic acid sequence encoding a PARG activity operably linked with the same molecule allowing targeting of the catalytic domain of PARP to the subcellular compartment or with a different molecule allowing targeting of PARG to the same or different subcellular compartment. Thus, it is possible to verify whether the presence or absence of PAR depends on the POI-PARP construct. Namely, in the presence of the PARP and the PARG construct in the same subcellular compartment will result in degradation of PAR, and, thus, will demonstrate that the PARP and the PARG construct are present in the same compartment.

Furthermore, a system, kit or assays provided for cytochemical or histochemical detection of a molecule of interest comprising i) a molecule having a poly-ADP-ribose-polymerase activity linked with a molecule able to bind directly or indirectly to the molecule of interest, or nucleic acid molecule encoding the same; ii) a substrate of the PARP enzyme optionally labelled to the marker moieties; iii) means for detecting poly-ADP-ribose formed by PARP activity. The referenced molecule having PARP activity linked with the molecule able to bind to the molecule of interest may be a molecule as described above, e.g. a single chain antibody linked with the catalytic domain of PARP or, alternatively, streptavidin or avidin as a fusion product with at least the catalytic domain of PARP.

The vector which may be used in the assay or system according to the present invention is e.g. an expression vector comprising the catalytic domain of PARP downstream of a multiple cloning site allowing introduction of the cDNA encoding the molecule of interest into the multiple cloning site. For example, upstream of the catalytic domain of PARP the nucleic acid sequence encoding the binding agent may be present, e.g. the single chain antibody, or alternatively, streptavidin or avidin. Of course, said expression vector may contain further nucleic acid sequence for expression or regulation of the vector system or the assay system.

In the system, kit or assay for cytochemical or histochemical detection of a molecule of interest, for example an antigen of interest, the molecule having a poly-ADP-ribose-polymerase activity linked with the molecule able to bind to the molecule of interest is preferably in form of a polypeptide. For example, said construct or fusion product is a fusion product of a single chain antibody or a polypeptide derived from an antibody linked with at least the catalytic domain of PARP. Alternatively, said fusion product is an antibody to which the PARP molecule is linked via covalent linkage or via e.g. the biotin-streptavidin system.

As expression vectors, commonly known expression vectors may be used, the linkage of PARP to antibodies may be achieved by known techniques. In addition, the preparation of fusion products composed of the polypeptides described herein is well known in the art.

In principle, the fusion construct, e.g. the antibody-PARP construct could be predecorated with PAR and the polymers be chemically modified with fluorophores. This would reduce the procedure of immunocytochemistry-immunohistochemistry, because only a single antibody incubation step would be required using the prelabled antibody. Importantly, even having only a single step, the signal would exceed that of conventional immunocytochemistry involving decorated secondary antibodies due to the attachment of detector molecules to the many polymer units, thus allowing manifold signal amplification.

Typically, NAD is used as a substrate for the PARP enzyme. NAD may be labelled in advance, e.g. with a fluorophore, a radioactive label or other known labels, e.g, biotin labelled NAD is available in the art. The PARP enzyme is able to polymerize the labelled NAD thus, generating PAR having a multitude of bound biotin. Said biotin can interact with streptavidin linked to a detector, like a fluorophore, enzyme activity or radioactivity. Thus, it is possible to amplify the signal due to the binding of a plurality of streptavidin to the PAR polymer. Alternatively, the PAR polymer may be in a form allowing chemical reaction with detector molecules. For example, PAR could be chemically modified in the ribose moieties in analogy with glycoside labelling known in the art. Also polymer specific antibodies are known as described in the examples below. Using these polymer specific antibodies labelled with e.g. a fluorophore, allows a sensitive and amplified detection of the MOI.

Figure 1 provides the immunodetection principle and improved sensitivity by enzymatically generated polymers. As shown in cases D and E showing embodiments of the present invention, the signal amplification by generation of numerous epitopes for the secondary antibody in D and E shows the superiority of the system towards common immunodetection techniques. Alternatively, the polymer itself may be labelled as described above.

While embodiment E represents a fusion product of the catalytic domain of PARP and a single chain antibody, embodiment D provides an example for an biotin labelled primary antibody wherein biotin labelled PARP is attached.

In the following, a detailed description of one embodiment of the present invention is provided.

The molecular architecture of PARP1 consists of an N-terminal DNA binding domain, an automodification domain and the C-terminal part containing the catalytic domain (Figure 2A). To verify the catalytic activity of the catalytic domain alone, a bacterially expressed, purified PARP1cd-6xHis construct harbouring the amino acid residues 652-1014 has been incubated in absence and presence of the substrate NAD₊. The PARP1cd generated PAR chains and automodified itself as detected by PAR Immunoblotting using 10H antibody (Figure 2B). No polymers were detectable in absence of NAD₊.

Fusion constructs of enhanced green fluorescent protein (EGFP) and PARP1cd (residues 572 to 1014) were generated and expressed in HelaS3 cells. Expression of this protein targeted to the mitochondrial matrix by an established targeting sequence (mitoPARP) led to PAR formation readily detectable by immunoblotting (Figure 2C). Strikingly, when the mitochondrial targeting sequence was omitted to generate a cytosolic protein (cytoPARP), no PAR formation could be observed (Figure 2C). Likewise, the control cytosolic and mitochondrial EGFP constructs lacking the PARP1cd moiety (cytoEGFP and mitoEGFP, respectively), did not lead to any detectable PAR formation (Figure 2C). We also closely inspected cells subjected to PAR immunocytochemistry after expressing the constructs for 24 h (Figure 2D). Again, PAR formation was detectable in cells that expressed the mitochondrial PARP1cd construct, whereas no signal was detectable in cells that expressed the construct lacking the MTS. These observations suggested that it was the subcellular localization, and not the nature of the PARP1cd fusion protein, which determined the capability to generate detectable PAR.

To test this hypothesis, proteins with known submitochondrial localization were used. Generally, the matrix targeting sequence resides in the N-terminus of a protein. Therefore, any additional sequence at the C-terminus of the protein should not alter its mitochondrial localization. Glutamate dehydrogenase (GDH) is a well established mitochondrial matrix protein and was therefore tested. As representative of the intermembrane space (IMS), we selected apoptosis inducing factor (AIF). This protein is reported to be N-terminally anchored in the inner mitochondrial membrane and to face towards the intermembrane space and possesses an N-terminal targeting sequence.

The open reading frames encoding these proteins were cloned into an eukaryotic expression vector that added a C-terminal FLAG-epitope to the encoded proteins. The according plasmids were transiently transfected into HeLaS3 cells and the resulting proteins detected by immunocytochemistry using an antibody specific for the FLAG epitope. Co-staining with MitoTracker®Red revealed the expected association with mitochondria. Next, vectors encoding fusion constructs of GDH and AIF with the catalytic domain of PARP1 were generated. Downstream of the PARP1cd moiety the proteins contained a myc-epitope at their C-termini. Following transient transfection of the vectors, myc-immunocytochemistry and co-staining with MitoTracker®Red revealed that these constructs showed a cellular distribution just as the FLAG constructs lacking the PARP1cd.

To test the capacity of these fusion proteins to generate PAR, cells have been transfected transiently with the PARP1cd fusion constructs and have been subjected to immunocytochemistry using antibodies specific for the myc epitope and PAR. The expression of the GDH-PARP1cd construct in HeLaS3 cells resulted in detectable formation of PAR that co-localized with the mycsignal of the protein (figure 3). This confirmed the previous findings of mitochondrial PAR generation by a matrix localized PARP1-construct (Niere *et al. supra*, 2008), using an authentic matrix protein. However, the cells that expressed the IMS-targeted AIF-PARP1cd construct were PAR-negative (figure 3). Only a minor portion of myc-positive cells showed PAR Immunoreactivity, however, only at the limit of detection.

As further evidence for the functionality of the system, a GDH-PARP1cd fusion construct was generated, which lacked the endogenous MTS encoded by the first 159 nucleotides of the open reading frame. Expression of this construct led to an accumulation of the protein in the cytosol. No detectable PAR were generated form this protein. In addition, targeted expression of AIF-PARP1cd to the mitochondrial matrix (mitoAIF-PARPcd) using the MTS of an authentic matrix protein resulted in detectable PAR formation in mitochondria.

The PAR immunoreactivity, which was exclusively observed for PARP1cd fusion proteins directed into the matrix, in fact relied on the enzymatic activity borne by PARP1cd. As exemplified for the GDH-PARP1cd fusion protein, PAR formation was no longer detectable upon treatment with the specific PARP inhibitor PJ34. Taken together, these results indicated that PARP1cd fusion may be generally used to establish whether a mitochondria-associated protein resides within the matrix.

Next, we sought to examine the subcellular distribution of key enzymes of all described NAD biosynthetic pathways in humans. Thus, expression vectors for a number of proteins as listed below have been generated. The phosphoribosyl transferases QPRT, NamPT and NAPRT as well as the two identified isoforms of nicotinamide riboside kinase all generate mononucleotide precursors of NAD, NAD synthetase converts the deamidated intermediate NAAD to NAD. As the only mitochondria-associated isoform, NMNAT3 was included in the Investigation. Furthermore, the cDNA sequence of NAPRT harbors a putative alternative start codon 72 bases downstream of the original start directly downstream of an internal Kozak consensus sequence (...CCACCATG...), which might be used by cells to express an alternative and possibly differentially located variant of NAPRT. Therefore, the truncated sequence (NAPRTΔ1-24) was included in the analyses. Despite existing reports for several of these proteins, their subcellular distribution is still ambiguous, since, for example, only a distinction between nuclear and cytoplasmic (i.e. nonnuclear) localization could be made.

First, the subcellular distribution of these proteins carrying a C-terminal FLAG tag was investigated. NAD synthetase displayed cytoplasmic distribution, whereas QPRT, both Nrk isoforms, NamPT and NAPRT (wildtype and truncated form) were detected both in the cytoplasm and the nucleus. None of these enzymes specifically colocalized with MitoTracker®Red. In contrast, NMNAT3 revealed a cellular distribution that followed the pattern of MitoTracker®Red, which confirms the previously described association with mitochondria.

Previously, NamPT was claimed to partially localize within mitochondria, a finding that easily might rest unnoticed in a classic immunocytochemical examination. Both NamPT and NAPRT are able to provide substrates for the mitochondrial NMNAT3-catalyzed reaction from permeable precursors and could, thus, establish a viable dinucleotide synthesis pathway within this organelle. Therefore, we generated vectors encoding NMNAT3, NamPT, and NAPRT as PARP1cd fusion proteins and transiently transfected them in HeLaS3 cells. Cells expressing NMNAT3-PARP1cd fusion proteins accumulated PAR within mitochondria. This established that NMNAT3 is indeed located within the mitochondrial matrix (figure 4). On the other hand, NamPT and NAPRT fusion proteins maintained their cytoplasmic distribution and did not give rise to any detectable PAR formation. Consequently, none of the two proteins is located within mitochondria (figure 4). The PARP1cd constructs seemed to be excluded from the nucleus. These observations conclusively demonstrate that NMNAT3 is indeed located within the matrix of mitochondria.

The possibility that structural aspects of the NamPT and NAPRT fusion proteins might prevent the PARP1cd portion from being active were considered. Therefore, said molecules have been endowed with the same artificial MTS that was previously used to target PARP1cd to the matrix (Niere et al 2008, supra). Transient transfection of cells with the mitoNamPT-PARP1cd and mitoNAPRT-PARP1cd construct resulted in an exclusive mitochondrial localization pattern of the proteins. Moreover, these constructs now generated detectable PAR as a result of their matrix localization. Thus, the functionality of the constructs with regard to the PARP activity was confirmed. In addition, this experiment further substantiated the suitability of the novel import assay.

Taken together, of the known enzymes involved in NAD biosynthetic pathways, only NMNAT3 could be conclusively localized to the mitochondrial matrix.

Herein, a novel assay has been developed to study the fine localization of mitochondrial proteins and identify those that reside in the mitochondrial matrix. The accurate identification of the subcellular localization of a protein is crucial in order to understand its physiological function. Indeed the analyses of the subcellular distribution of enzymes involved in NAD biosynthesis have established that among all known proteins and isoforms only NMNAT3 is located within the mitochondrial matrix. Given the large size and important role of the mitochondrial NAD pool as well as the existence of several independent routes of NAD biosynthesis, this is a rather unexpected conclusion. All proteins analyzed in this study were localized to the cytoplasm, while some displayed an additional nuclear localization. Thus according to the presence of enzymes in these compartments, the presence and conversions of intermediates would be expected. Although never truly demonstrated, it is assumed that all the metabolites including NAD itself can freely permeate between the nucleus and the cytosol (but not the mitochondria).

The novel assay is based on a biochemical approach and relies on the observation that upon expression of the catalytic activity of PARP1, the accumulation of its product, PAR, is only detectable when the protein is present in the mitochondrial matrix. This system differs from other approaches by its simplicity, as it merely requires standard procedures of molecular and cellular biology and conventional fluorescence microscopy. The visualization of an enzymatic activity strengthens the system by reducing the possibility of false positive results, which may occur in other techniques, such as subcellular fractionation and protease treatment import assays on isolated mitochondria. Moreover, the availability of PARP1 inhibitors allows the system to be easily controlled. The application of this assay on both matrix (GDH) and intermembrane space (AIF) mitochondrial proteins clearly distinguished between differential suborganellar distributions of the analyte proteins. This stands in contrast to classic immunodetection of a protein of interest and co-staining with mitochondrial markers, which allow only for conclusions concerning an association with mitochondrial structures. The restrictions of such co-staining experiments were aimed to overcome with the present approach. The reason for the distinct PAR signal from matrix and IMS remains concealed, but restricted availability of the non-bound substrate NAD+ outside of the matrix may be a contributing factor. With regard to cytosolically localized fusion proteins, a fast PAR turnover by cytosolic PARG could result in undetectable signals. In principle, this system is suitable to serve as a universal tool for establishing matrix localization of any given candidate proteins. Even membrane proteins embedded in the inner mitochondrial membrane may be examined with regard to their topology and orientation, provided the C-terminus protrudes on either side of the membrane.

This novel localization assay has been applied with the intention to elucidate subcellular and suborganellar distribution of those enzymatic activities that are important for, and may be directly involved in the maintenance of the mitochondrial NAD pool. An in depth analysis is required in order to determine which possible precursors might have to enter mitochondria to be finally converted to NAD in the matrix. The establishment of this separate NAD pool is of special interest due to the major redox energy metabolism and NAD degrading reactions in this compartment, involved in processes such as insulin secretion and intraorganellar protein acetylation.

### Material and Methods

### Chemicals, reagents and media

Unless otherwise specified, all chemicals and reagents were of analytical grade and purchased from Sigma-Aldrich and Merck. Reagents other than fetal bovine serum (Biochrom) were from Cambrex Cooperation, Nunc or Invitrogen/Gibco. PJ34 was obtained from Calblochem. Anti-FLAG and anti-β-Tubulin antibodies were from Sigma-Aldrich, the rabbit anti-PAR antibody from Alexis Biochemicals and the chicken anti-myo antibody from Invitrogen. Mouse anti-myc antibody (9E10) and mouse anti-PAR antibody (10H) were from hybridoma cell culture supernatants. Fluorescent-conjugated secondary antibodies were of highly cross-adsorbed quality from Invitrogen/Molecular Probes. ECL reagents and HRP conjugated goat anti-mouse /goat anti-rabbit antibodies were from Pierce or GE healthcare/Amersham Biosciences. DNA modifying and restriction enzymes were purchased from Fermentas, New England Biolabs or TaKaRa, oligonucleotide synthesis was done by Sigma-Aldrich.

### Cloning and generation of eukaryotic expression vectors

The DNA sequence encoding the C-terminal catalytic domain of PARP1 (amino acids (aa) 572-1014) along with a C-terminal myc-epitope was amplified from a pre-existing vector (Niere *et al*., supra 2008) and ligated intopcDNA3.1(+) (Invitrogen) via *EcoRI*/*Xbal* sites. Subsequently, the open reading frames (ORFs) of the proteins of interest were amplified and introduced into the generated pcDNA3,1(+)-PARP1cd vector via *KpnI*/*EcoRI* restriction sites. In order to verify the localization of the proteins of interest by indirect immunocytochemistry, their corresponding cDNA sequences were inserted into pFLAG-CMV 5a (Sigma). In addition, the ORFs of some proteins were ligated into pCMV mito myc (Invitrogen) using the Sall restriction site. All cloned cDNAs were amplified using *Pfu* DNA polymerase. Recognition sites for restriction enzymes and the Kozak sequence were included in the primer sequences.

### Cell culture

HeLaS3 cells were cultivated in Ham's F12 medium supplemented with 10% (v/v) FCS and penicillin/streptomycin. 293 cells were cultivated in Dulbecco's modified Eagle's medium and supplemented with 10% (v/v) FCS, 2 mM glutamine and penicillin/streptomycin. Transient transfection of eukaryotic cells was performed for 24-48h using Effectene reagent (Qiagen) according to the recommendations of the manufacturer.

### Immunocytochemistry

Cells grown on cover slips were fixed with ice cold 4% (v/v) formaldehyde in PBS for 45 min and subsequently permeabilized for 15 min using 0.5% (v/v) Triton X-100 in PBS. In some experiments, cells were treated with 0.2 µM MitoTracker®Red CMXRos (Invitrogen) in full medium for 30 min prior to fixation. After blocking with complete medium for 1 h at room temperature (RT), cells were incubated with primary antibodies diluted in complete medium for ≥ 1 h at RT or overnight at 4°C. Cells were washed once with 0.1% (v/v) Triton X-100 in PBS and three times with PBS followed by a 1 h incubation at RT with secondary antibody diluted 1:1000 in complete medium. After staining the nuclei with DAPI, the cells were washed three times with PBS and the cover slips were mounted onto slides. Samples were analyzed on a Leica DMI 6000B microscope (Leica Microsystems).

### Protein determination, SDS-PAGE and Western Biot analysis

Protein concentration determination of cell lysates prepared with 20 mM Tris/HCl pH 7.4, 150 mM NaCl, 2 % SDS and 1 mM EDTA as lysis buffer was done using a bicinchoninic acid kit (Pierce). Sodium dodecyl sulfate poly acrylamide gel electrophoresis (SDS-PAGE) and immunoblotting were carried out according to standard procedures; enhanced chemiluminescence was used for detection.

## Claims

1. A method for cytochemical or histochemical detection of a molecule of interest comprising the steps of:
a. providing a molecule having a poly-ADP-ribose-polymerase (PARP) activity linked with a molecule able to bind to the molecule of interest, or nucleid acids encoding the same;
b. allowing binding of the molecule of a.) to the molecule of interest;
c. providing a substrate of the PARP enzyme and generation of poly-ADP-ribose (PAR) by enzymatic reaction of the PARP enzyme;
d. detection of PAR.

2. An invitro method for screening for compounds altering the amounts of NAD in subcellular compartments comprising the steps of:
a. providing a fusion construct of a catalytic moiety of PARP and a moiety directing the catalytic moiety of PARP to a predetermined subcellular compartment;
b. providing a cell system containing the compounds of interest;
c. introducing the fusion construct into the cell system;
d. determining the amount of PAR formed in the subcellular compartment of in cells of the cell systems.

3. An invitro method for determining the spatial distribution of a molecule of interest, comprising the steps of:
a. providing a fusion construct comprising the catalytic domain of PARP and the molecule of interest;
b. introducing the fusion construct into a cell;
c. determining the localisation of the fusion construct based on the presence of PAR.

4. A method for cytochemical or histochemical detection of a molecule of interest comprising the steps of:
a. providing a molecule having a PARP activity linked with a molecule able to bind to the molecule of interest;
b. allowing binding of the molecule of a.) with the molecule of interest;
c. detection of PAR.

5. The method according to any one of claims 1 or 4 wherein the molecule able to bind to the molecule of interest is a polypeptide derived from an antibody.

6. The method according to claim 5 wherein the polypeptide is a single chain antibody.

7. The method according to any one of the preceding claims wherein the substrate of the PARP is NAD, optionally labelled with a label or marker moiety.

8. The method according to any one of the preceding claims wherein the detection of PAR is effected by immunocytochemical or immunohystochemical methods, in particular, antibody based methods whereby said antibodies are labelled with a marker moiety conjugated.

9. The method according to claim 2 or 3 wherein the fusion construct is in form of an expression vector.

10. The method according to any one of the preceding claims wherein the substrate for the PARP enzyme is provided exogenously.

11. The method according to any one of the preceding claims wherein an inhibitor of the poly-ADP-ribose glycohydrolase is present.

12. The method according to claim 2 for identifying compounds for inducing adoptosis in cells.

13. The method according to claim 2 for identifying compounds for the treatment of cancer.

14. Kit for analysing subcellular localisation of metabolits comprising
- a vector comprising a nucleic acid sequence encoding the catalytic domain of PARP (seq. ID. no.1) operably linked with a molecule allowing targeting of the catalytic domain of PARP to the subcellular compartment,
- optionally, a vector comprising a nucleic acid sequence encoding PARG operably linked with the same molecule allowing targeting of the catalyltic domain of PARP to the subcellular compartment or with a different molecule allowing targeting of PARG to the same or different subcellular compartment and
- means for detection of PAR.

15. Kit for cytochemical or histochemical detection of a molecule of interest comprising i) a molecule having a poly-ADP-ribose-polymerase activity (seq. ID. no.2) linked with a molecule able to bind to the molecule of interest or nucleid acid molecule encoding the same; ii) a substrate of the PARP enzyme optionally labelled with a marker moiety; iii) means for detecting poly-ADP-ribose.

16. The use of the PARP enzyme in cytochemical or histochemical detection of a molecule of interest.

## Patentansprüche

1. Verfahren zur zytochemischen oder histochemischen Detektion eines interessierenden Moleküls umfassend die Schritte:
a) Bereitstellen eines Moleküls mit einer Poly-ADP-Ribose-Polymerase (PARP) Aktivität verknüpft mit einem Molekül, das an das interessierende Molekül binden kann oder hierfür kodierende Nukleinsäuren;
b) Ermöglichen der Bindung des Moleküls von a) an das interessierende Molekül;
c) Bereitstellen eines Substrats des PARP-Enzyms und Bildung von Poly-ADP-Ribose (PAR) durch enzymatische Reaktionen des PARP-Enzyms;
d) Nachweis von PAR.

2. Ein In-vitro-Verfahren zum Screenen von Verbindungen, die die Menge an NAD in subzellulären Kompartimenten verändern, umfassend die Schritte:
a) Bereitstellen eines Fusionskonstrukts einer katalytischen PARP-Einheit und einer Einheit, die die katalytische Einheit von PARP zu einem vorbestimmten subzellulären Kompartiment leitet;
b) Bereitstellen eines Zellsystems enthalten die interessierenden Verbindungen;
c) Einbringen des Fusionskonstrukts in das Zellsystem;
d) Bestimmen der Menge an gebildeten PAR in der subzellulären Kompartimente der Zellen des Zellsystems.

3. In-vitro-Verfahren zur Bestimmung der örtlichen Verteilung eines interessierenden Moleküls, umfassend die Schritte:
a) Bereitstellen eines Fusionskonstrukts umfassend die katalytische Domäne von PARP und des interessierenden Moleküls;
b) Einbringen des Fusionskonstrukts in eine Zelle;
c) Bestimmen der Lokalisierung des Fusionskonstrukts auf Basis des Vorhandenseins von PAR.

4. Verfahren zum zytochemischen oder histochemischen Nachweis eines interessierenden Moleküls, umfassend die Schritte:
a) Bereitstellen eines Moleküls mit einer PARP-Aktivität verknüpft mit einem Molekül, das an das interessierende Molekül binden kann;
b) Ermöglichen einer Bindung des Moleküls von a) mit dem interessierenden Molekül;
c) Nachweis von PAR.

5. Verfahren nach einem der Ansprüche 1 bis 4, wobei das Molekül, das an das interessierende Molekül binden kann, ein Polypeptid ist, das von einem Antikörper stammt.

6. Verfahren nach Anspruch 5, wobei das Polypeptid ein Einzelkettenantikörper ist.

7. Verfahren nach einem der vorherigen Ansprüche, wobei das Substrat von PARP NAD ist, ggf. markiert mit einem Label oder einer Markereinheit.

8. Verfahren nach einem der vorherigen Ansprüche, wobei der Nachweis von PAR erreicht wird durch immunzytochemische oder immunhistochemische Verfahren, insbesondere basierend auf Antikörper, wobei die Antikörper mit einer konjugierten Markeeinheit markiert sind.

9. Verfahren nach einem der Ansprüche 2 oder 3, wobei das Fusionskonstrukt in Form eines Expressionsvektors ist.

10. Verfahren nach einem der vorherigen Ansprüche, wobei das Substrat für das PARP-Enzym exogen bereitgestellt wird.

11. Verfahren nach einem der vorherigen Ansprüche, wobei ein Inhibitor der Poly-ADP-Ribose Glycohydrolase anwesend ist.

12. Verfahren nach Anspruch 2 zur Identifizierung von Verbindungen, die Apoptose in Zellen induzieren.

13. Verfahren nach Anspruch 2 zur Identifizierung von Verbindungen zur Behandlung von Krebs.

14. Kit zur Analyse der subzellulären Lokalisierung von Metaboliten umfassend:
a) Ein Vektor umfassend eine Nukleinsäuresequenz für die katalytische PARP Domäne operativ verknüpft mit einem Molekül, das ein Targeting der katalytischen PARP Domäne zu einen subzellulären Kompartiment erlaubt, ggf. einen Vektor umfassend eine Nukleinsäuresequenz für PARG operativ verknüpft mit dem gleichen Molekül, das ein im Targeting der katalytischen PARP Domäne zu einem subzellulären Kompartiment erlaubt oder mit einem ganz unterschiedlichen Molekül, das ein Targeting von PARG zum gleichen oder einem unterschiedlichen subzellulären Kompartiment erlaubt und Mittel zum Nachweis von PAR.

15. Kit zur zytochemischen oder histochemischen Detektion eines interessierenden Moleküls umfassend
i) ein Molekül mit Poly-ADP-Ribose-Polymerase Aktivität verknüpft mit einem Molekül, das an das interessierende Molekül binden kann oder dafür kodierende Nukleinsäuremoleküle;
ii) ein Substrat des PARP-Enzyms ggf. markiert mit einer Markereinheit;
iii) Mittel zum Nachweis der Poly-ADP-Ribose.

16. Verwendung des PARP Enzyms in zytochemischen oder histochemischen Nachweis eines interessierenden Moleküls.

## Revendications

1. Procédé de détection cytochimique ou histochimique d'une molécule d'intérêt, comprenant les étapes de :
a. mise à disposition d'une molécule ayant une activité de poly-ADP-ribose-polymérase (PARP) liée à une molécule apte à se lier à la molécule d'intérêt, ou des acides nucléiques codant pour elle ;
b. amenée de la molécule a.) à se lier à la molécule d'intérêt ;
c. mise à disposition d'un substrat de l'enzyme PARP, et production de poly-ADP-ribose (PAR) par réaction enzymatique de l'enzyme PARP ;
d. détection du PAR.

2. Procédé *in vitro* pour le criblage des composés altérant la quantité de NAD dans des compartiments sous-cellulaires, comprenant les étapes de :
a. mise à disposition d'une construction de fusion d'un fragment catalytique de PARP et d'un fragment dirigeant le fragment catalytique de PARP vers un compartiment sous-cellulaire prédéterminé ;
b. mise à disposition d'un système cellulaire contenant les composés d'intérêt ;
c. introduction de la construction de fusion dans le système cellulaire ;
d. détermination de la quantité de PAR formée dans le compartiment sous-cellulaire dans des cellules des systèmes cellulaires.

3. Procédé *in vitro* de détermination de la distribution spatiale d'une molécule d'intérêt, comprenant les étapes de :
a. mise à disposition d'une construction de fusion comprenant le domaine catalytique de PARP et la molécule d'intérêt ;
b. introduction de la construction de fusion dans une cellule ;
c. détermination de la localisation de la construction de fusion, en se fondant sur la présence de PAR.

4. Procédé de détection cytochimique ou histochimique d'une molécule d'intérêt, comprenant les étapes de :
a. mise à disposition d'une molécule ayant une activité de PARP, liée à une molécule apte à se lier à la molécule d'intérêt ;
b. amenée de la molécule a.) à se lier à la molécule d'intérêt ;
c. détection de PAR.

5. Procédé selon l'une quelconque des revendications 1 à 4, dans lequel la molécule apte à se lier à la molécule d'intérêt est un polypeptide qui dérive d'un anticorps.

6. Procédé selon la revendication 5, dans lequel le polypeptide est un anticorps monocaténaire.

7. Procédé selon l'une quelconque des revendications précédentes, dans lequel le substrat du PARP est le NAD, en option marqué par un marqueur ou un fragment marqueur.

8. Procédé selon l'une quelconque des revendications précédentes, dans lequel la détection de PAR est réalisée par des techniques immunocytochimiques ou immunohistochimiques, en particulier des techniques à base d'anticorps, lesdits anticorps étant marqués par un fragment marqueur conjugué.

9. Procédé selon la revendication 2 ou 3, dans lequel la construction de fusion se présente sous forme d'un vecteur d'expression.

10. Procédé selon l'une quelconque des revendications précédentes, dans lequel le substrat pour l'enzyme PARP est mis à disposition d'une manière exogène.

11. Procédé selon l'une quelconque des revendications précédentes, dans lequel un inhibiteur de la poly-ADP-ribose-glycohydrolase est présent.

12. Procédé selon la revendication 2, pour identifier les composés destinés à provoquer une apoptose dans des cellules.

13. Procédé selon la revendication 2, pour identifier les composés destinés au traitement du cancer.

14. Trousse destinée à analyser une localisation sous-cellulaire de métabolites, comprenant
- un vecteur comprenant une séquence d'acide nucléique codant pour le domaine catalytique de PARP, lié d'une manière opérationnelle à une molécule permettant le ciblage du domaine catalytique de PARP vers le compartiment sous-cellulaire,
- en option, un vecteur comprenant une séquence d'acide nucléique codant pour le PARG liée d'une manière opérationnelle à la même molécule que celle permettant le ciblage du domaine catalytique de PARP au compartiment sous-cellulaire, ou à une molécule différente, permettant le ciblage de PARG vers le même compartiment sous-cellulaire ou vers un compartiment sous-cellulaire différent, et
- des moyens pour détecter le PAR.

15. Trousse pour la détection cytochimique ou histochimique d'une molécule d'intérêt, comprenant i) une molécule ayant une activité de poly-ADP-ribose-polymérase, liée à une molécule apte à se lier à la molécule d'intérêt ou à une molécule d'acide nucléique codant pour cette dernière ; ii) un substrat de l'enzyme PARP, en option marqué par un fragment marqueur ; iii) des moyens pour détecter la poly-ADP-ribose.

16. Utilisation de l'enzyme PARP dans le cadre de la détection cytochimique ou histochimique d'une molécule d'intérêt.
